# EUROPEAN PATENT SPECIFICATION

(11) **EP 4 316 565 B1**
(45) Date of publication and mention of the grant of the patent: **02.12.2026**
(21) Application number: 22774058.6
(22) Date of filing: 10.03.2022
(51) Int. Cl.: A61M 25/01, A61B 5/00, B05D 1/26

(54) **METHOD FOR SEALING DISTAL END OF OCT CATHETER**
VERFAHREN ZUM ABDICHTEN DES DISTALEN ENDES EINES OCT-KATHETERS
PROCÉDÉ POUR SCELLER L'EXTRÉMITÉ DISTALE D'UN CATHÉTER OCT

(30) Priority: 25.03.2021 CN 202110321785
(43) Date of publication of application: 07.02.2024
(73) Proprietor: Suzhou Microport Argus Medical Corp, Suzhou, Jiangsu 215007 (CN)
(72) Inventor: FU, Yuqi, Suzhou, Jiangsu 215007 (CN); WU, Zhenying, Suzhou, Jiangsu 215007 (CN); ZHANG, Lintao, Suzhou, Jiangsu 215007 (CN)
(74) Representative: karo IP
(86) International application number: PCT/CN2022/080176
(87) International publication number: WO 2022/199396

(56) References cited:
- CN-A- 104 053 471
- CN-A- 105 188 833
- CN-A- 108 289 690
- CN-A- 112 495 704
- CN-A- 113 083 633
- CN-U- 208 524 844
- US-A- 5 496 346
- US-A1- 2009 018 393
- US-A1- 2011 077 463
- US-A1- 2014 180 076
- US-A1- 2016 058 413
- US-A1- 2016 354 111

## Description

### FIELD OF THE INVENTION

The present application relates to the technical field of optical interference tomography, and specifically relates to a method for sealing the distal end of an OCT catheter.

### BACKGROUND OF THE INVENTION

Optical coherence tomography (OCT) is a new type of tomographic imaging technology that has developed rapidly in recent years and has the most promising prospects, especially biopsy and imaging of biological tissues. It has been tried to be applied in clinical diagnosis of cardiology, ophthalmology, dentistry and dermatology. It is another major technological breakthrough after X-CT and MRI technology. In recent years, it has been developed rapidly. The optical interference tomography system based on this technology can enable people to obtain ultra-high-definition images with spatial resolution on the order of microns. When the imaging catheter used for optical interference tomography imaging system scanning is used to seal the distal end of the catheter, in order to seal the catheter, a section of ultraviolet adhesive needs to be injected into the guide wire hole and cured. The existing operation generally adopts the following method: the operator uses a dispensing machine, and after dispensing and molding at one time, uses an ultraviolet lamp to irradiate and cure.

The existing adhesive dispensing method has the following disadvantages: after one dispensing, due to the surface tension of the liquid, after the ultraviolet adhesive is cured and molded, the end faces at both ends are almost at right angles. When the OCT catheter is actually used, the distal end needs to pass through a 0.356 mm (0.014") guide wire, the right-angled end face of the ultraviolet adhesive can easily obstruct the guide wire, making it difficult for the guide wire to pass through, affecting the doctor's feel and delaying the operation time. CN 108 289 690 A describes an endoscope treatment instrument.

### SUMMARY OF THE INVENTION

In view of the above problems, the present application provides a method for sealing the distal end of an OCT catheter as set out in the claims. During surgery, the guide wire can easily pass through the catheter and is not easily obstructed, which makes it easy for doctors to operate during surgery and saves operation time.

**Furthermore, an** OCT catheter is disclosed having a recessed portion, the recessed portion includes a filling section and a recessed hole section, and the radial size of the filling section gradually decreases in the direction close to the recessed hole section in the axial direction until it connects with the recessed hole section, the filling section is filled with a first sealant, and the end of the first sealant connected to the recessed hole section is the first end, the end away from the recessed hole section is the second end, a second sealant is connected to the first end of the first sealant, and the second sealant has an inclined surface, and the inclined surface is at least partially located within the recessed hole section.

The recessed hole section may comprise a recessed hole and a first space located below the recessed hole, the recessed hole has a bottom end and a top end, and the bottom end of the recessed hole is connected with the first end of the first sealant, and the top end of the recessed hole is away from the first sealant.

**A** radial size of the first space may gradually increase in an axial direction from the bottom end to the top end.

The second sealant may be at least partially located within the first space.

**T**he first end surface and the second end surface of the first sealant may both be perpendicular to the tube wall.

**T**he included angle between the inclined surface and the tube wall below the recessed hole may not exceed 60°.

**T**he included angle between the inclined surface and the tube wall below the recessed hole may be 45°.

**T**he OCT catheter may be prepared by the method described in any one of claims 1-10.

The method for sealing the distal end of an OCT catheter provided by this application is simple to operate. The guide wire of the prepared OCT catheter can easily pass through the recessed hole during the operation and is not easily obstructed by the first sealant and the second sealant, which makes it easy for doctors to operate during surgery and saves operation time.

### BRIEF DESCRIPTION OF THE DRAWINGS

The accompanying drawings are used for better understanding of the present application, and do not constitute an improper limitation of the present application.
wherein:
Figure 1 is a schematic structural diagram of the OCT catheter.
Figure 2 is a schematic structural diagram of the OCT catheter when used.

### Description of reference numerals

1-OCT catheter, 2-first sealant, 3-recessed hole, 4-first space, 5-second sealant, 6- inclined surface, 7-guide wire.

### DETAIL DESCRIPTION OF THE INVENTION

The following describes the exemplary embodiments of the present application, including various details of the embodiments of the present application to facilitate understanding, and they should be considered as exemplary only. Also, descriptions of well-known functions and constructions are omitted from the following description for clarity and conciseness. The present application provides a method for sealing the distal end of an OCT catheter, comprising the following steps:
Step 1: dispensing an adhesive for the first time, passing an adhesive dispensing needle through the OCT catheter from a recessed hole section to dispense an adhesive in the direction of a filling section, the adhesive entering the filling section inside the OCT catheter,
Step 2: curing for the first time, irradiating the filling section after the first dispensing by using an ultraviolet lamp, and curing to form the first sealant;
Step 3: dispensing an adhesive for the second time, enabling the adhesive dispensing needle to be close to a solidified end surface after the first dispensing to dispense an adhesive;
Step 4: curing for the second time, placing the position after the second dispensing under the ultraviolet lamp for irradiation, curing to form the second sealant having an inclined surface, and completing sealing the distal end of the OCT catheter after curing.

The adhesive used in adhesive dispensing for the first time and adhesive dispensing for the second time is ultraviolet adhesive; for example, Dymax 1201-M-SC, Dymax 1202-M-SC, and Dymax 1204-M-SC.

The ultraviolet adhesive used for adhesive dispensing for the first time is in the filling section. Due to the gravitational force of the upper and lower tube walls in the filling section on the ultraviolet adhesive, the ultraviolet adhesive will not flow freely in the filling section, so that after curing, both ends of the first sealant are perpendicular to the upper and lower pipe walls or tangents of the upper and lower pipe walls in the filling section that are connected to the first sealant.

In this application, the dispensing pressure of adhesive dispensing for the first time is 6-8kg/cm², preferably 7kg/cm², and the time of adhesive dispensing is 0.15-0.25s, preferably 0.2s;
The dispensing pressure of adhesive dispensing for the first time can be 6kg/cm², 6.1kg/cm², 6.2kg/cm², 6.3kg/cm², 6.4kg/cm², 6.5kg/cm², 6.6kg/cm², 6.7kg/cm², 6.8kg/cm², 6.9kg/cm², 7kg/cm², 7.1kg/cm², 7.2kg/cm², 7.3kg/cm², 7.4kg/cm², 7.5kg/cm², 7.6kg/cm², 7.7kg/cm², 7.8kg/cm², 7.9kg/cm², 8kg/cm².

The time of adhesive dispensing for the first time can be 0.15s, 0.16s, 0.17s, 0.18s, 0.19s, 0.2s, 0.21s, 0.22s, 0.23s, 0.24s, 0.25s.

The dispensing pressure of adhesive dispensing for the second time is 1-3kg/cm², preferably 2kg/cm², and the time of adhesive dispensing is 0.15-0.25s, preferably 0.2s.

The dispensing pressure of adhesive dispensing for the second time can be 1kg/cm², 1.1kg/cm², 1.2kg/cm², 1.3kg/cm², 1.4kg/cm², 1.5kg/cm², 1.6kg/cm², 1.7kg/cm², 1.8kg/cm², 1.9kg/cm², 2kg/cm², 2.1kg/cm², 2.2kg/cm², 2.3kg/cm², 2.4kg/cm², 2.5kg/cm², 2.6kg/cm², 2.7kg/cm², 2.8kg/cm², 2.9kg/cm², 3kg/cm².

The time of adhesive dispensing for the second time can be 0.15s, 0.16s, 0.17s, 0.18s, 0.19s, 0.2s, 0.21s, 0.22s, 0.23s, 0.24s, 0.25s.

The first curing time is 5-15s, preferably 8s; the second curing time is 5-15s, preferably 8s.

The ratio of the first dispensing amount to the second dispensing amount is (2-8):1.

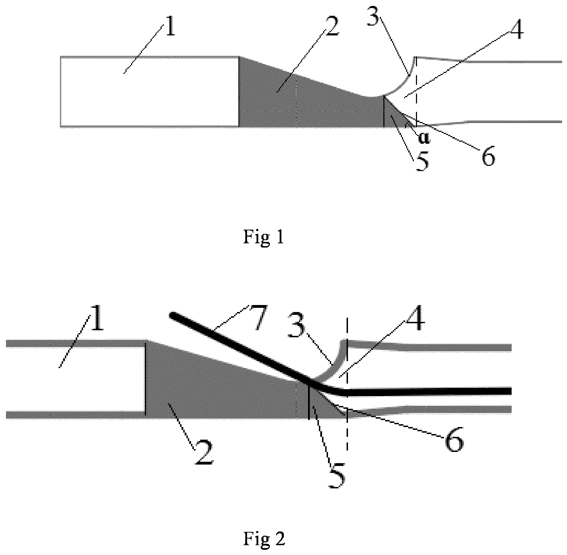

As shown in FIG. 1, the OCT catheter 1 has a recessed portion, which comprises a filling section and a recessed hole section. The radial size of the filling section gradually decreases in the direction axially close to the recessed hole section, until it connects with the recessed hole section, the filling section is filled with a first sealant 2, and the end of the first sealant 2 connected to the recessed hole section is the first end, the end far from the recessed hole section is the second end, the first end of the first sealant 2 is connected with a second sealant 5, and the second sealant 5 has an inclined surface 6, and the inclined surface 6 is at least partially located within the recessed hole section.

The first sealant 2 fills the filling section, and the first sealant 2 and the second sealant 5 form a sealing layer to seal the distal end of the OCT catheter 1. Moreover, due to the inclined surface 6 of the second sealant 5, during the operation, the guide wire 7 can easily pass through the recessed hole section along the inclined surface 6, and the guide wire 7 will not be easily obstructed by the second sealant 5, thereby improving the efficiency of doctors' surgeries.

The recessed hole section comprises a recessed hole 3 and a first space 4 located below the recessed hole 3, the recessed hole 3 has a bottom end and a top end, and the bottom end of the recessed hole 3 is connected to the first end of the first sealant 2, and the top end of the recessed hole 3 is away from the first sealant 2. The radial size of the first space 4 gradually increases in the axial direction from the bottom end to the top end. The radial size of the first space 4 at the bottom end of the recessed hole 3 is consistent with the radial size of the first end of the first sealant 2, and the radial size of the first space 4 at the top end of the recessed hole 3 is consistent with the inner diameter of the OCT catheter 1.

The second sealant 5 is at least partially located within the first space 4.

In a specific configuration, the inclined surface 6 of the second sealant 5 can extend through the first space 4 to the distal end of the OCT catheter 1.

In another specific configuration, the inclined surface 6 of the second sealant 5 is located within the first space 4.

The included angle α between the inclined surface 6 and the tube wall below the recessed hole 3 does not exceed 60°, that is, the included angle α between the inclined surface 6 and the tube wall below the first space 4 does not exceed 60°, preferably 45°. In actual situations, the included angle α can be 60°, 59°, 58°, 57°, 56°, 55°, 54°, 53°, 52°, 51°, 50°, 49°, 48°, 47°, 46°, 45°, 44°, 43°, 42°, 41°, 40°, 39°, 38°, 37°, 36°, 35°, 34°, 33°, 32°, 31 °, 30°, etc.

As shown in Figure 2, when the OCT catheter is used, the guide wire 7 passes through the distal end of the OCT catheter 1. When the guide wire 7 reaches the first space 4, the guide wire 7 reaches the recessed hole 3 along the inclined surface 6 of the second sealant 5 and passes through the recessed hole 3. The first sealant 2 and the second sealant 5 not only separate the distal and proximal ends of the catheter, and the inclined surface 6 of the second sealant 5 is conducive to the penetration of the guide wire 7, so that the doctor can improve the operating efficiency during surgery.

Although the embodiments of the present application have been described above, the present application is not limited to the above-mentioned specific embodiments and application fields, and the above-mentioned specific embodiments are only illustrative, instructive, rather than restrictive.

## Claims

1. A method for sealing the distal end of an OCT catheter (1), wherein it comprises the following steps:
dispensing an adhesive for the first time, passing an adhesive dispensing needle through the OCT catheter (1) from a recessed hole section to dispense an adhesive in the direction of a filling section, the adhesive entering the filling section inside the OCT catheter (1),
curing for the first time, **characterized in that**, irradiating the filling section after the first dispensing by using an ultraviolet lamp, and curing to form a first sealant (2);
dispensing an adhesive for the second time, enabling the adhesive dispensing needle to be close to a solidified end surface after the first dispensing time to dispense an adhesive;
curing for the second time, placing the position after the second dispensing under the ultraviolet lamp for irradiation, curing to form the second sealant (5) having an inclined surface (6), and completing sealing the distal end of the OCT catheter (1) after curing.

2. The method for sealing the distal end of an OCT catheter (1) according to claim 1, wherein the adhesive used in the adhesive dispensing for the first time and the adhesive dispensing for the second time is ultraviolet adhesive.

3. The method for sealing the distal end of an OCT catheter (1) according to claim 1, wherein the dispensing pressure of the adhesive dispensing for the first time is 6-8kg/cm², preferably 7kg/cm², and the time of adhesive dispensing is 0.15-0.25s, preferably 0.2s;
the dispensing pressure of the adhesive dispensing for the second time is 1-3kg/cm², preferably 2kg/cm², and the time of adhesive dispensing is 0.15-0.25s, preferably 0.2s.

4. The method for sealing the distal end of an OCT catheter (1) according to claim 1, wherein the time of curing for the first time is 5-15s, preferably 8s; the time of curing for the second time is 5-15s, preferably 8s.

5. The method for sealing the distal end of an OCT catheter (1) according to claim 1, wherein the end of the first sealant (2) connected to the recessed hole section is the first end, and the end far from the recessed hole section is the second end, the second sealant (5) is connected to the first end of the first sealant (2), and the second sealant (5) has an inclined surface (6), and the inclined surface (6) is at least partially located within the recessed hole section.

6. The method for sealing the distal end of an OCT catheter (1) according to claim 5, wherein the recessed hole section comprises a recessed hole (3) and a first space (4) located below the recessed hole (3), and the recessed hole (3) has a bottom end and a top end, the bottom end of the recessed hole (3) is connected to the first end of the first sealant (2), and the top end of the recessed hole (3) is away from the first sealant (2).

7. The method for sealing the distal end of an OCT catheter (1) according to claim 6, wherein the radial size of the first space (4) gradually increases in the axial direction from the bottom end to the top end; and/or wherein the second sealant (5) is at least partially located within the first space (4).

8. The method for sealing the distal end of an OCT catheter (1) according to claim 5, wherein the first end surface and the second end surface of the first sealant (2) are both perpendicular to the tube wall of the OCT catheter (1).

9. The method for sealing the distal end of an OCT catheter (1) according to claim 6, wherein the included angle between the inclined surface (6) and the tube wall of the OCT catheter (1) below the recessed hole (3) does not exceed 60°, preferably 45°.

## Patentansprüche

1. Verfahren zum Abdichten des distalen Endes eines OCT-Katheters (1), wobei es die folgenden Schritte umfasst:
erste Klebstoff-Abgabe, wobei eine Klebstoff-Abgabenadel von einem vertieften Lochabschnitt aus durch den OCT-Katheter (1) geführt wird, um Klebstoff in Richtung eines Füllbereichs abzugeben, wobei der Klebstoff in den Füllbereich im Inneren des OCT-Katheters (1) gelangt,
erstes Aushärten, **dadurch gekennzeichnet, dass** der Füllabschnitt nach der ersten Abgabe unter Verwendung einer UV-Lampe bestrahlt wird und ausgehärtet wird, um ein erstes Dichtungsmittel (2) zu bilden;
zweite Klebstoff-Abgabe, wobei die Klebstoff-Abgabenadel nach der ersten Abgabe in die Nähe einer ausgehärteten Endfläche gebracht wird, um Klebstoff abzugeben;
zweites Aushärten, wobei die Stelle nach der zweiten Abgabe unter die UV-Lampe zur Bestrahlung gebracht wird, und Aushärten, um das zweite Dichtungsmittel (5) mit einer schrägen Oberfläche (6) zu bilden, sowie das Fertigstellen des Abdichtens des distalen Endes des OCT-Katheters (1) nach dem Aushärten.

2. Verfahren zum Abdichten des distalen Endes eines OCT-Katheters (1) nach Anspruch 1, wobei der bei der ersten und der zweiten Klebstoff-Abgabe verwendete Klebstoff ein UV-Klebstoff ist.

3. Verfahren zum Abdichten des distalen Endes eines OCT-Katheters (1) nach Anspruch 1, wobei der Abgabedruck bei der ersten Klebstoff-Abgabe 6-8 kg/cm², vorzugsweise 7 kg/cm², beträgt, und die Zeit der Klebstoff-Abgabe 0,15-0,25 s, vorzugsweise 0,2 s, beträgt;
und wobei der Abgabedruck bei der zweiten Klebstoff-Abgabe 1-3 kg/cm², vorzugsweise 2 kg/cm², beträgt, und die Zeit der Klebstoff-Abgabe 0,15-0,25 s, vorzugsweise 0,2 s, beträgt.

4. Verfahren zum Abdichten des distalen Endes eines OCT-Katheters (1) nach Anspruch 1, wobei die Zeit des ersten Aushärtens 5-15 s, vorzugsweise 8 s, beträgt; und wobei die Zeit des zweiten Aushärtens 5-15 s, vorzugsweise 8 s, beträgt.

5. Verfahren zum Abdichten des distalen Endes eines OCT-Katheters (1) gemäß Anspruch 1, wobei das mit dem vertieften Lochabschnitt verbundene Ende des ersten Dichtungsmittels (2) das erste Ende ist, und das vom vertieften Lochabschnitt entfernte Ende das zweite Ende ist, das zweite Dichtungsmittel (5) mit dem ersten Ende des ersten Dichtungsmittel (2) verbunden ist, und das zweite Dichtungsmittel (5) eine schräge Oberfläche (6) aufweist, wobei sich die schräge Oberfläche (6) zumindest teilweise innerhalb des vertieften Lochabschnitts befindet.

6. Verfahren zum Abdichten des distalen Endes eines OCT-Katheters (1) nach Anspruch 5, wobei der vertiefte Lochabschnitt ein vertieftes Loch (3) und einen ersten Raum (4) umfasst, der sich unterhalb des vertieften Lochs (3) befindet, und das vertiefte Loch (3) ein unteres Ende und ein oberes Ende aufweist, wobei das untere Ende des vertieften Lochs (3) mit dem ersten Ende des ersten Dichtungsmittel (2) verbunden ist und das obere Ende des vertieften Lochs (3) vom ersten Dichtungsmittel (2) entfernt ist.

7. Verfahren zum Abdichten des distalen Endes eines OCT-Katheters (1) nach Anspruch 6, wobei sich die radiale Größe des ersten Raums (4) in axialer Richtung vom unteren Ende zum oberen Ende hin allmählich vergrößert; und/oder wobei sich das zweite Dichtungsmittel (5) zumindest teilweise innerhalb des ersten Raums (4) befindet.

8. Verfahren zum Abdichten des distalen Endes eines OCT-Katheters (1) gemäß Anspruch 5, wobei die erste Endfläche und die zweite Endfläche des ersten Dichtungsmittels (2) beide senkrecht zur Rohrwand des OCT-Katheters (1) stehen.

9. Verfahren zum Abdichten des distalen Endes eines OCT-Katheters (1) nach Anspruch 6, wobei der zwischen der schrägen Oberfläche (6) und der Rohrwand des OCT-Katheters (1) unterhalb des vertieften Lochs (3) gebildete Winkel 60°, vorzugsweise 45°, nicht überschreitet.

## Revendications

1. Procédé de scellement de l'extrémité distale d'un cathéter OCT (1), lequel comprend les étapes suivantes :
distribution d'un adhésif pour la première fois, en faisant passer une aiguille de distribution d'adhésif à travers le cathéter OCT (1) depuis une section de trou en retrait pour distribuer un adhésif en direction d'une section de remplissage, l'adhésif pénétrant dans la section de remplissage à l'intérieur du cathéter OCT (1), polymérisation pour la première fois, **caractérisé par** l'irradiation de la section de remplissage après la première distribution au moyen d'une lampe ultraviolette, et la polymérisation pour former un premier produit de scellement (2) ;
distribution d'un adhésif pour la seconde fois, en amenant l'aiguille de distribution d'adhésif à proximité d'une surface d'extrémité solidifiée après la distribution pour la première fois pour distribuer un adhésif ;
polymérisation pour la seconde fois, en plaçant la position après la seconde distribution sous la lampe ultraviolette en vue de l'irradiation, en polymérisant pour former le second produit de scellement (5) présentant une surface inclinée (6), et en achevant le scellement de l'extrémité distale du cathéter OCT (1) après la polymérisation.

2. Procédé de scellement de l'extrémité distale d'un cathéter OCT (1) selon la revendication 1, dans lequel l'adhésif utilisé dans la distribution d'adhésif pour la première fois et la distribution d'adhésif pour la seconde fois est un adhésif polymérisable aux ultraviolets.

3. Procédé de scellement de l'extrémité distale d'un cathéter OCT (1) selon la revendication 1, dans lequel la pression de distribution de la distribution d'adhésif pour la première fois est de 6 à 8 kg/cm², de préférence de 7 kg/cm², et le temps de distribution d'adhésif est de 0,15 à 0,25 s, de préférence de 0,2 s ;
la pression de distribution de la distribution d'adhésif pour la seconde fois est de 1 à 3 kg/cm², de préférence de 2 kg/cm², et le temps de distribution d'adhésif est de 0,15 à 0,25 s, de préférence de 0,2 s.

4. Procédé de scellement de l'extrémité distale d'un cathéter OCT (1) selon la revendication 1, dans lequel le temps de polymérisation pour la première fois est de 5 à 15 s, de préférence de 8 s ; le temps de polymérisation pour la seconde fois est de 5 à 15 s, de préférence de 8 s.

5. Procédé de scellement de l'extrémité distale d'un cathéter OCT (1) selon la revendication 1, dans lequel l'extrémité du premier produit de scellement (2) reliée à la section de trou en retrait est la première extrémité, et l'extrémité éloignée de la section de trou en retrait est la seconde extrémité, le second produit de scellement (5) est relié à la première extrémité du premier produit de scellement (2), et le second produit de scellement (5) présente une surface inclinée (6), et la surface inclinée (6) est au moins partiellement située à l'intérieur de la section de trou en retrait.

6. Procédé de scellement de l'extrémité distale d'un cathéter OCT (1) selon la revendication 5, dans lequel la section de trou en retrait comprend un trou en retrait (3) et un premier espace (4) situé au-dessous du trou en retrait (3), et le trou en retrait (3) présente une extrémité inférieure et une extrémité supérieure, l'extrémité inférieure du trou en retrait (3) est reliée à la première extrémité du premier produit de scellement (2), et l'extrémité supérieure du trou en retrait (3) est éloignée du premier produit de scellement (2).

7. Procédé de scellement de l'extrémité distale d'un cathéter OCT (1) selon la revendication 6, dans lequel la dimension radiale du premier espace (4) augmente progressivement dans la direction axiale de l'extrémité inférieure vers l'extrémité supérieure ; et/ou dans lequel le second produit de scellement (5) est au moins partiellement situé à l'intérieur du premier espace (4).

8. Procédé de scellement de l'extrémité distale d'un cathéter OCT (1) selon la revendication 5, dans lequel la première surface d'extrémité et la seconde surface d'extrémité du premier produit de scellement (2) sont toutes deux perpendiculaires à la paroi du tube du cathéter OCT (1).

9. Procédé de scellement de l'extrémité distale d'un cathéter OCT (1) selon la revendication 6, dans lequel l'angle formé entre la surface inclinée (6) et la paroi du tube du cathéter OCT (1) au-dessous du trou en retrait (3) ne dépasse pas 60°, de préférence 45°.
